# EUROPEAN PATENT APPLICATION

(11) **EP 2 835 099 A1**
(43) Date of publication of application: **11.02.2015**
(21) Application number: 14171014.5
(22) Date of filing: 03.06.2014
(51) Int. Cl.: A61B 3/113, G06F 3/01

(54) **Perusing determination device and perusing determination method**

(30) Priority: 06.08.2013 JP 2013163632
(71) Applicant: FUJITSU LIMITED, Kawasaki-shi, Kanagawa 211-8588 (JP)
(72) Inventor: Taguchi, Akinori, Kawasaki-shi, Kanagawa 211-8588 (JP)
(74) Representative: Wilding, Frances Ward

(57) **Abstract**

A perusing determination device, includes a computer processor, the device includes, an eye tracking unit configured to detect a movement direction of line-of-sight of a user who reads a document; a return movement counting unit configured to count a first number of times in which the movement direction of the line-of-sight is reversed from a direction in which the document is to be read; and a determination unit configured to determine whether or not the user peruses the document, based on the first number and a second number of times in which the movement direction of the line-of-sight is reversed that is to be satisfied at a time of perusing and is defined based on a feature of the document.

## Description

### FIELD

The embodiments discussed herein are related to, for example, a perusing determination device, a perusing determination method, and a perusing determination program.

### BACKGROUND

Up until recently, various pieces of information including character information have been created as digital data. For example, there has been increased an opportunity to read an electronic document such as an electronic book and a document file through a monitor. In addition, there also has been increased an opportunity to read on a monitor, for example, a document that is desired to be perused and includes a content to be grasped correctly such as a business e-mail, a procedure manual, and an instruction manual.

On the other hand, recently, a technology has been desired by which it may be automatically determined whether or not a user peruses an electronic document. For example, in Japanese Laid-open Patent Publication No. 2006-107048, a method of detecting order in which a user has referred to parts that are included in an electronic document, based on a direction of the user's gaze has been discussed.

However, in the related art, it may be determined whether or not the user has referred to the parts that are included in the document in correct order, but it is difficult to accurately determine whether or not the user has perused the electronic document (whether or not the user has not skimmed the electronic document).

It is desirable to provide a perusing determination device, a perusing determination method, and a perusing determination program by which it may be accurately determined whether or not a user has perused a document.

### SUMMARY

In accordance with an embodiment of an aspect, a perusing determination device, includes a computer processor, the device includes, an eye tracking unit configured to detect a movement direction of line-of-sight of a user who reads a document; a return movement counting unit configured to count a first number of times in which the movement direction of the line-of-sight is reversed from a direction in which the document is to be read; and a determination unit configured to determine whether or not the user peruses the document, based on the first number and a second number of times in which the movement direction of the line-of-sight is reversed that is to be satisfied at a time of perusing and is defined based on a feature of the document.

The object and advantages of the invention will be realized and attained by means of the elements and combinations particularly pointed out in the claims. It is to be understood that both the foregoing general description and the following detailed description are exemplary and explanatory and are not restrictive of the invention, as claimed.

In the perusing determination device, the perusing determination method, and the perusing determination program according to the embodiments discussed herein, it may be accurately determined whether or not a user has perused a document

### BRIEF DESCRIPTION OF DRAWINGS

These and/or other embodiments of aspects and advantages will become apparent and more readily appreciated from the following description of the embodiments, taken in conjunction, by way of example only, with the accompanying drawing of which:
FIG. 1 is a schematic diagram illustrating a structure of an information processing system according to a first embodiment;
FIG. 2 is a diagram illustrating a hardware structure of an information processing device illustrated in FIG. 1;
FIG. 3 is a functional block diagram illustrating the information processing device illustrated in FIG. 1;
FIG. 4 is a diagram illustrating an electronic document that is displayed on a display in the first embodiment;
FIG. 5 is a flowchart illustrating processing of the information processing device according to the first embodiment;
FIG. 6 is a flowchart illustrating processing of an information processing device according to a second embodiment;
FIG. 7 is a flowchart illustrating processing of an information processing device according to a third embodiment;
FIG. 8A is a diagram illustrating a reversal DB that is used in the third embodiment, and FIG. 8B is a diagram illustrating a reference reversal DB that is used in the third embodiment;
FIG. 9A is a diagram illustrating a threshold value table that is used in a fourth embodiment, and FIG. 9B is a first flowchart illustrating processing of an information processing device according to the fourth embodiment.
FIG. 10 is a second flowchart illustrating the processing of the information processing device according to the fourth embodiment;
FIG. 11 is a diagram illustrating a modification; and
FIGs. 12A and 12B are diagrams illustrating modifications.

### DESCRIPTION OF EMBODIMENTS

### [First embodiment]

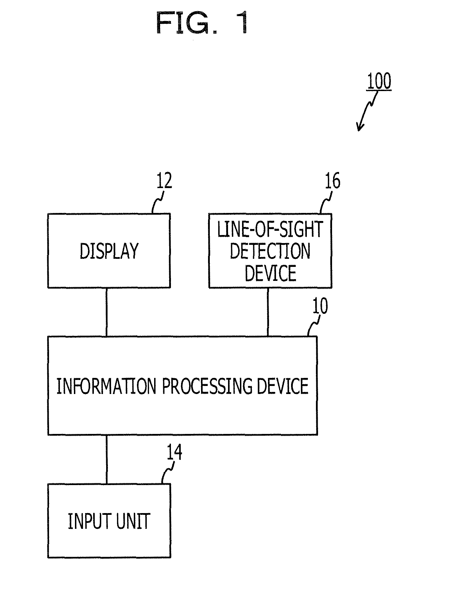

An information processing system according to a first embodiment is described below with reference to FIGs. 1 to 5. In FIG. 1, a structure of an information processing system 100 according to the first embodiment is schematically illustrated.

The information processing system 100 includes an information processing device 10 as a perusing determination device, a display 12, an input unit 14, and a line-of-sight detection device 16. The information processing device 10 displays a content and a document on the display 12 in response to an instruction from a user, and executes processing and the like of determining whether or not the user has perused the content and the document, by obtaining a detection result of the line-of-sight detection device 16. The detailed structure and processing of the information processing device 10 are described later.

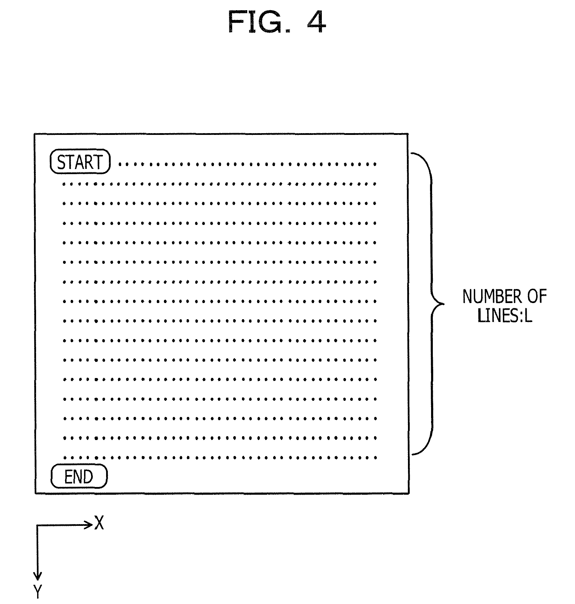

The display 12 includes a liquid crystal display, and displays the content, the document, and the like in response to an instruction from the information processing device 10. In the embodiment, as an example, a case is described in which an electronic document having a layout as illustrated in FIG. 4 is displayed on the display 12. The electronic document in FIG. 4 is a document that includes L horizontal lines, and is, for example, a document that is desirable for perusing such as a business e-mail, a procedure manual, an instruction manual. In addition, it is assumed that a start button is arranged at the upper left of the electronic document, and an end button is arranged at the lower left of the electronic document. The display 12 may include a plurality of displays (multi-display).

The input unit 14 includes a keyboard, a mouse, a touch-screen, and the like, and receives an input from the user.

The line-of-sight detection device 16 includes a near-infrared lighting (light emitting diode: LED) and a camera, and is a device that detects a line-of-sight direction in a noncontact manner by a corneal reflection method. The line-of-sight detection device 16 is provided in a part of or in the vicinity of the display 12, and may detect a position in the display 12 to which the line-of-sight of the user who sees the display 12 is directed. The line-of-sight detection device 16 may detect the line-of-sight direction of the user by a method other than the corneal reflection method.

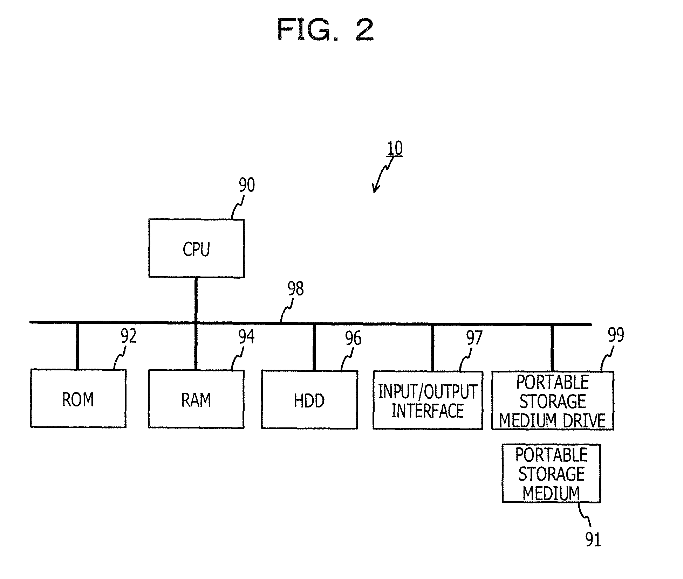

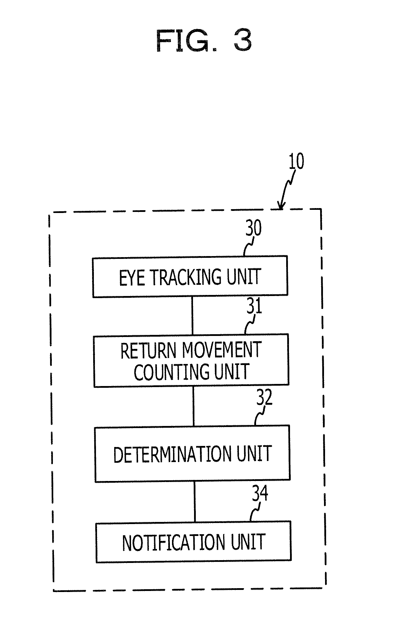

In FIG. 2, a hardware structure of the information processing device 10 is illustrated. As illustrated in FIG. 2, the information processing device 10 includes a central processing unit (CPU) 90, a read-only memory (ROM) 92, a random access memory (RAM) 94, a storage unit (here, a hard disk drive (HDD)) 96, an input/output interface 97, and a portable storage medium drive 99, and each of the configuration units in the information processing device 10 is connected to a bus 98. In the information processing device 10, a program that includes a perusing determination program and is stored in the ROM 92 or the HDD 96, or a program that includes the perusing determination program and is read from a portable storage medium 91 by the portable storage medium drive 99 is executed by the CPU 90, so that a function of each unit in FIG. 3 is achieved.

In FIG. 3, a functional block diagram of the information processing device 10 is illustrated. As illustrated in FIG. 3, in the information processing device 10, the CPU 90 executes the program, so that functions as an eye tracking unit 30, a return movement counting unit 31, a determination unit 32, and a notification unit 34 are achieved.

The eye tracking unit 30 calculates time variation in a line-of-sight position that is detected in the line-of-sight detection device 16. That is, the eye tracking unit 30 detects a direction in which the line-of-sight of the user is moved on the electronic document (FIG. 4). The return movement counting unit 31 counts the number (i.e., a first number) of times in which the movement direction of the line-of-sight of the user is reversed from a direction in which the electronic document is to be read (left to right in FIG. 4).

The determination unit 32 determines whether or not the user has perused the electronic document, based on the counting result of the return movement counting unit 31. Here, in the embodiment, the determination unit 32 determines, through the line-of-sight of the user, whether or not the user has perused the electronic document, based on the number of return sweeps or the number of backward-directed eye movements that include regression and refixation and are specific movements of the line-of-sight when the user peruses a character string. The notification unit 34 notifies the user of the determination result of the determination unit 32.

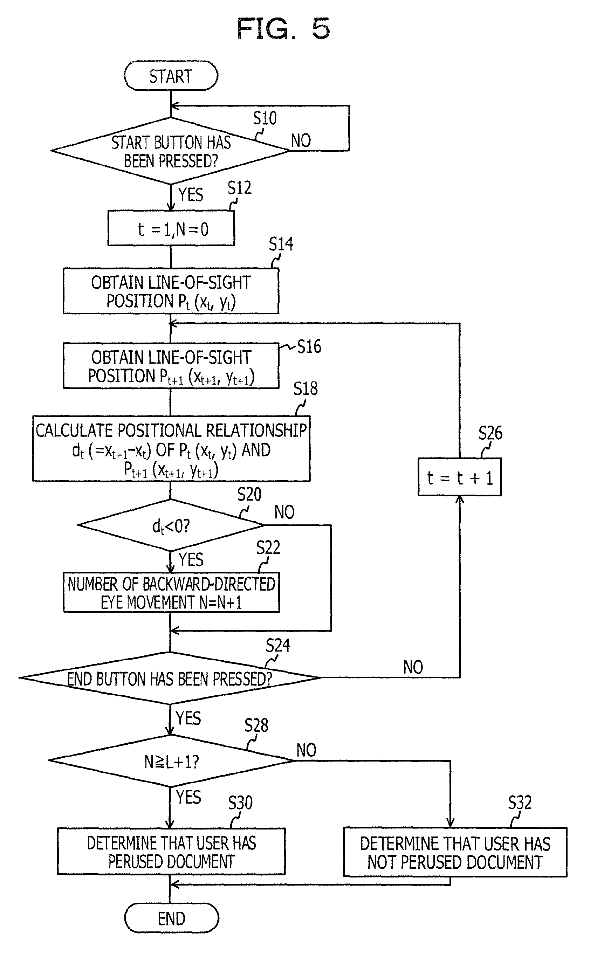

Processing by the information processing device 10 according to the first embodiment is described in detail below with reference to the flowchart of FIG. 5. As a precondition that the processing of FIG. 5 is started, it is assumed that the electronic document in FIG. 4 is displayed on the display 12, and the user sees the display 12.

In the processing in FIG. 5, first, in Step S10, the eye tracking unit 30 waits until the user presses the start button through the input unit 14. In Step S10, the eye tracking unit 30 may wait until the user performs a certain trigger operation instead of the pressing of the start button. As the certain trigger operation, there is an operation in which the user fixes the eyes on a certain position and an operation that is related on the line-of-sight such as wink. In addition, the eye tracking unit 30 may wait until utterance of certain voice by the user is detected using the microphone. The eye tracking unit 30 is started up after an input from the user through the input unit 14 is received, and may wait until a certain operation is detected in Step S10. Alternatively, the eye tracking unit 30 may typically be running. When the eye tracking unit 30 is typically running, prediction of a line-of-sight position using previous line-of-sight information allows a case in which line-of-sight is not detected due to wink at a time of the trigger operation, and the like to be dealt with.

When "Yes" is determined in Step S10, in next Step S12, the eye tracking unit 30 sets a value t that indicates a serial number of a line-of-sight position at 1, and sets a value N that indicates the number of backward-directed eye movements at 0.

After that, in Step S14, the eye tracking unit 30 obtains a line-of-sight position Pₜ (xₜ, yₜ) (here, P₁ (x₁, y₁)) on the screen of the display 12 from the line-of-sight detection device 16.

After that, in Step S16, the eye tracking unit 30 obtains a line-of-sight position Pₜ₊₁ (Xₜ₊₁, yₜ₊₁) (here, P₂ (x₂, y₂)) on the screen of the display 12 from the line-of-sight detection device 16. After Step S14, it is assumed that Step S16 is executed after a certain time period has elapsed (for example, a time period that is desired to complete return sweep by the user (about tens of milliseconds)). As the certain time period, generally, a frame rate of the camera in the line-of-sight detection device 16 may be employed.

After that, in Step S18, the eye tracking unit 30 calculates a positional relationship "dₜ=Xₜ₊₁-Xₜ" that is related to an X-axis direction (direction in which the document is to be read) of Pₜ (xₜ, yₜ) and Pₜ₊₁ (Xₜ₊₁, yₜ₊₁). Here, the eye tracking unit 30 calculates a positional relationship "d₁=P₂ (X₂, y₂)-P₁ (x₁, y₁)". When the line-of-sight position moves forward during sentence reading, the positional relationship dₜ becomes a positive value. When the line-of-sight position moves backward during sentence reading (here, a backward-directed eye movement includes a regression, a refixation, and a return sweep), the positional relationship dₜ becomes a negative value. That is, the eye tracking unit 30 also detects a movement direction of the line-of-sight by calculating the positional relationship dt. In Step S18, a distance between two points for the X-axis direction is calculated as the positional relationship, but a vector or the like may be calculated instead of the distance.

After that, in Step S20, the return movement counting unit 31 determines whether or not the positional relationship dₜ is less than 0 (negative). When "No" is determined in Step S20, that is, when the line-of-sight position is moved in the direction in which the electronic document is to be read, the flow proceeds to Step S24. In addition, when "Yes" is determined in Step S20, that is, the movement direction of the line-of-sight position is reversed from the direction in which the electronic document is to be read, the flow proceeds to Step S22.

When the flow proceeds to Step S22, the return movement counting unit 31 increments the number of backward-directed eye movements N by 1 (N=N+1), and the flow proceeds to Step S24.

When the flow proceeds to Step S24, the return movement counting unit 31 determines whether or not the end button has been pressed. When "No" is determined in Step S24, the flow proceeds to Step S26. Similar to Step S10, in Step S24, it may be determined whether or not a further certain input is performed, or it may be determined whether or not information by which completion of reading of the document may be estimated is obtained. As the information by which completion of reading of the document may be estimated, for example, information that indicates that the line-of-sight position exists near the end position of the electronic document, information on the number of lines that are read by the user, which is estimated from the movement of the line-of-sight, and the like are assumed.

In Step S26, the eye tracking unit 30 increments the value t by 1 (t=t+1). After that, the eye tracking unit 30 and the return movement counting unit 31 count the number of backward-directed eye movements N by repeating the processing and determination in Steps S16 to S26. In addition, when "Yes" is determined in Step S24, the flow proceeds to Step S28.

When the flow proceeds to Step S28, the determination unit 32 determines whether or not "N" is a threshold value (L+1) or more. Here, the threshold value is set at "L+1", because it may be estimated that the user peruses the document when the number of backward-directed eye movements is larger than the number of lines (number of line changes), that is, when a backward-directed eye movement expect return sweep is performed more than once. The threshold value may be "L+a" ("a" is an integer other than "1"). The value a may be defined based on a feature of the document such as the length of the whole electronic document, the number of characters for one line, and the number of lines. When "Yes" is determined in Step S28, the flow proceeds to Step S30, and when "No" is determined in Step S28, the flow proceeds to Step S32.

When the flow proceeds to Step S30, that is, when "N" is "L+1" or more, the determination unit 32 determines that the user has perused the electronic document. In addition, when the flow proceeds to Step S32, the determination unit 32 determines that the user has not perused the electronic document.

The determination result of the determination unit 32 is transmitted to the notification unit 34. The notification unit 34 may display the determination result on the display 12. Instead of the display, for example, voice may be output using a speaker that is not illustrated. In addition, the determination result of the determination unit 32 may be used for processing other than notification. For example, display control may be performed depending on the determination result of the determination unit 32 so that a next content or electronic document is not allowed to be displayed when the user has not perused the electronic document. In addition, for example, control may be performed depending on the determination result of the determination unit 32 so that a certain button is not allowed to be pressed. In addition, the determination results of the determination unit 32 may be collected, and statistics may be taken whether or not the user who uses the content peruses the electronic document.

As described above, in the first embodiment, the threshold value that is used in Step S28 indicates the number of backward-directed eye movements that is to be satisfied at the time of perusing, which is defined by the feature of the electronic document.

As described above in detail, in the first embodiment, the eye tracking unit 30 detects a movement direction of the line-of-sight of the user who reads the electronic document, and the return movement counting unit 31 counts the number of times in which the movement direction of the line-of-sight of the user is reversed from the direction in which the electronic document is to be read. In addition, the determination unit 32 determines whether or not the user has perused the electronic document, based on the counting result of the return movement counting unit 31 and the number (i.e., a second number) of backward-directed eye movements that is to be satisfied at the time of perusing, which is defined by the feature of the electronic document (in the first embodiment, the threshold value L+1 or more). As a result, in the first embodiment, determination may be performed in which whether or not a backward-directed eye movement occurs through the line-of-sight of the user is considered, so that it may be accurately determined whether or not the user has perused the electronic document.

In addition, in the first embodiment, the number of backward-directed eye movements that is to be satisfied at the time of perusing (threshold value) is a value that is defined based on the number of lines (L) that are included in the document, so that an appropriate threshold value may be set in which the number of return sweeps of the user is considered.

There is a device error in the line-of-sight detection device 16, and it is probable that a backward-directed eye movement is recognized erroneously due to the device error. Here, it is known that the erroneous recognition occurs in accordance with certain probability distribution independently of a read character string. Therefore, the return movement counting unit 31 may determine whether or not the backward-directed eye movement occurs, by considering a tendency of a document that is a determination target (occurrence probability of erroneous recognition for each time period and read position) in addition to a positional relationship between coordinates.

### [Second embodiment]

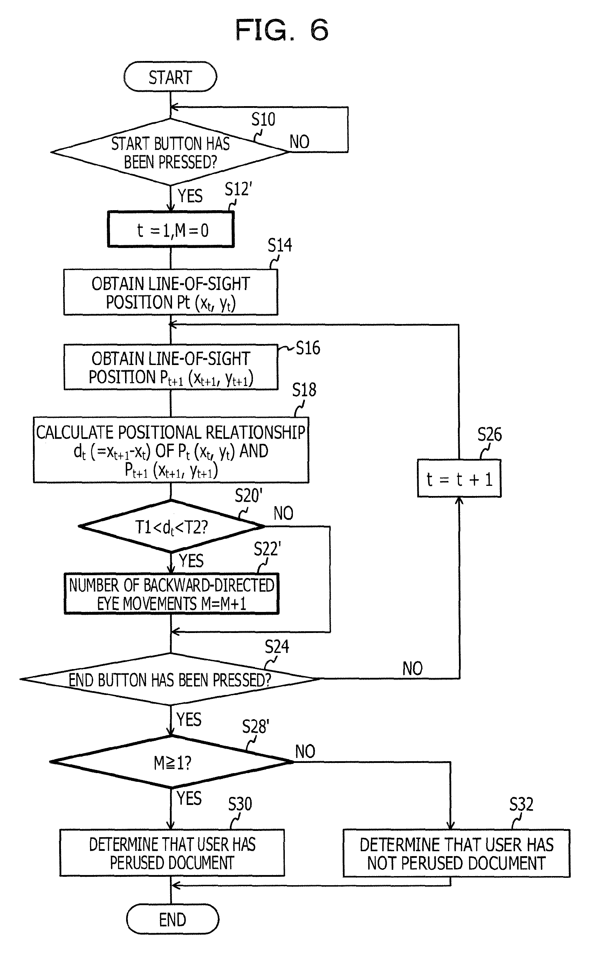

A second embodiment is described below with reference to FIG. 6. A system structure, a device structure, and the like according to the second embodiment are similar to those of the first embodiment, but processing of the return movement counting unit 31 is different from the first embodiment.

In FIG. 6, processing of an information processing device 10 according to the second embodiment is illustrated by a flowchart. In FIG. 6, processing that is different from the above-described processing in first embodiment is illustrated by the heavy line. In the second embodiment, the number of backward-directed eye movements is counted, and whether or not the user has perused the electronic document is determined based on the counting result.

In the processing of FIG. 6, when "Yes" is determined in Step S10, in next Step S12', the eye tracking unit 30 sets, at 1, a value t that indicates a serial number of a line-of-sight position, and sets, at 0, a value M that indicates the number of backward-directed eye movements.

After that, in Steps S14 to S18, similar to the above-described processing in the first embodiment, the eye tracking unit 30 identifies line-of-sight positions Pₜ (xₜ, yₜ) and Pₜ₊₁ (xₜ₊₁, yₜ₊₁), and calculates a positional relationship dt (=xₜ₊₁-xₜ) that is related to the X-axis direction (direction in which the document is to be read).

After that, in Step S20', the return movement counting unit 31 determines whether or not the positional relationship dₜ is larger than a first threshold value T1 and smaller than a second threshold value T2. Here, the first threshold value T1 is a negative value, and for example, may employ a value such as "(80% of the length of a line)×(-1)". The second threshold value T2 is also a negative value, and may employ a value such as "(value that is equal to or more than an error range of the line-of-sight detection device 16 (error range that is related to the X-axis direction))×(-1)". As the second threshold value T2, "(size for the X-axis direction of the certain number of characters of an electronic document (for example, two characters))×(-1)" or "(size for the X-axis direction of a range in which characters are allowed to be recognized once, which is estimated from a visual field characteristic (for example, a central visual field)/2)×(-1)" may be employed. The first threshold value T1 and the second threshold value T2 may be set based on a further reference. When a condition of "T1<dₜ<T2" is satisfied, it is indicated that a backward-directed eye movement occurs.

When "No" is determined in Step S20', that is, when a backward-directed eye movement does not occur, the flow proceeds to Step S24, and when "Yes" is determined in Step S20', the flow proceeds to Step S22'. When the flow proceeds to Step S22', the return movement counting unit 31 increments the number of backward-directed eye movements M by 1 (M=M+1). In addition, when "No" is determined in Step S24, the flow returns to Step S16 through Step S26, and the processing and the determination in Steps S16 to S26 are repeated until "Yes" is determined in Step S24.

After that, when "Yes" is determined in Step S24, the flow proceeds to Step S28', and the determination unit 32 determines whether "M" is 1 or more, that is, whether the number of backward-directed eye movements is 1 or more. When "Yes" is determined in Step S28', the flow proceeds to Step S30, and the determination unit 32 determines that the user has perused the electronic document. In addition, when "No" is determined in Step S28', the flow proceeds to Step S32, and the determination unit 32 determines that the user has not perused the electronic document.

As described above, in the second embodiment, a device error of the line-of-sight detection device 16 is considered, so that it may be further accurately determined whether or the user has perused the electronic document. For example, when the line-of-sight stops, there is a case in which it is determined that the line-of-sight moves in a direction opposite to the direction in which the electronic document is to be read due to the drive error of the line-of-sight detection device 16, but it may be accurately determined whether or not the user has perused the electronic document by removing such a device error.

When a device error of the line-of-sight detection device 16 is small, the return movement counting unit 31 may determine whether or not the positional relationship dₜ is larger than the first threshold value T1 and smaller than 0 in Step S20'.

### [Third embodiment]

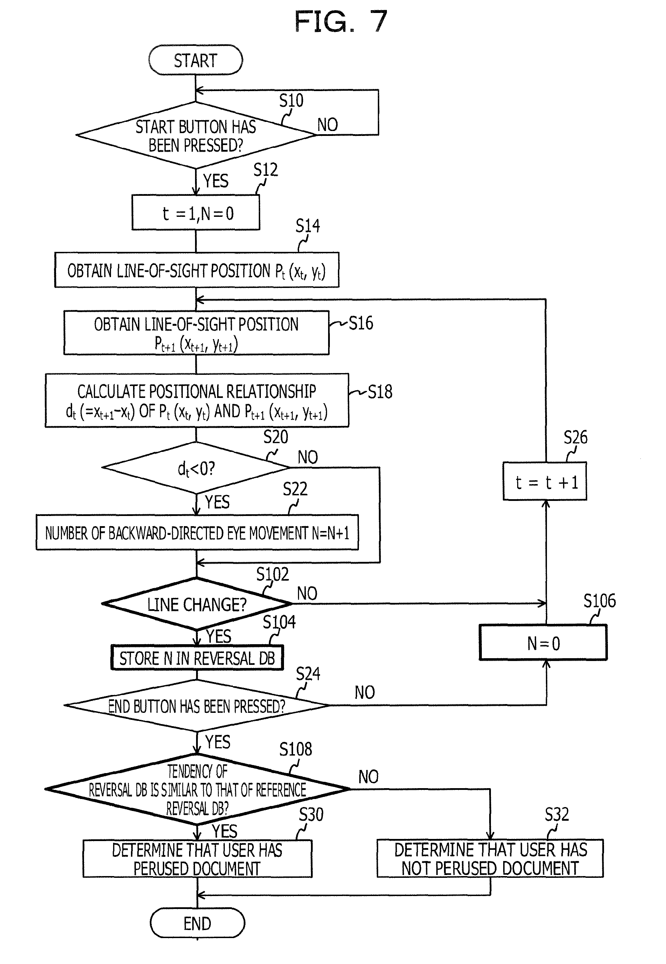

A third embodiment is described below with reference to FIGs. 7 to 8B. A structure and the like of an information processing system according to the third embodiment are similar to those of the above-described information processing system 100 according to the first embodiment, but a method of determining whether or not the user has perused an electronic document is different from the first embodiment.

FIG. 7 is a flowchart illustrating processing of an information processing device 10 according to the third embodiment. In FIG. 7, processing and determination that are different from the processing in the first embodiment (FIG. 5) are illustrated by the heavy line.

In the processing of FIG. 7, processing in Steps S10 to S22 is executed similar to the processing of the first embodiment. In addition, when the flow proceeds to Step S102, the return movement counting unit 31 determines whether or not a return sweep has occurred. For example, the return movement counting unit 31 determines whether or not the most recently-calculated positional relationship dₜ is a value that corresponds to the number of return sweeps. It may be determined whether or not the positional relationship dₜ is a value that correspond to the number of return sweeps based on whether or not "dₜ<T1" is satisfied (here, it is assumed that "T1" is identical to the first threshold value in the second embodiment). Alternatively, it is conceivable that X coordinates at the line-of-sight position is included in a certain range (left end of the electronic document) after the return sweep. Thus, in Step S102, when the X coordinates at the line-of-sight position is included in the certain range, it may be determined that a return sweep has occurred.

When "No" is determined in Step S102, the flow proceeds to Step S26. When "Yes" is determined in Step S102, the flow proceeds to Step S104.

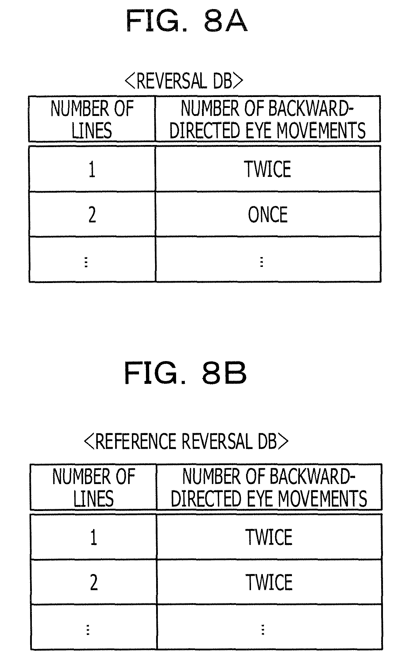

When the flow proceeds to Step S104, that is, when there has occurred the return sweep, the return movement counting unit 31 stores "N" in a reversal DB. Here, it is assumed that the reversal DB have a data structure as illustrated in FIG. 8A. For example, when a backward-directed eye movement of the user has not occurred even once while the user reads the electronic document for one line, "1" is stored in a field of the number of backward-directed eye movements in the reversal DB. When a backward-directed eye movement of the user has occurred n times while the user reads for one line, "1+n" is stored in the field of the number of backward-directed eye movements in the reversal DB.

Returning to FIG. 7, in next Step S24, the return movement counting unit 31 determines whether or not the end button has been pressed. When "No" is determined in Step S24, the flow proceeds to Step S106. In Step S106, the return movement counting unit 31 returns "N" to 0, and the flow proceeds to Step S26. After that, until "Yes" is determined in Step S24, the processing in Step S16 and the subsequent steps are repeated, and the number of backward-directed eye movements in each line is stored in the reversal DB.

In addition, when "Yes" is determined in Step S24, the flow proceeds to Step S108. When the flow proceeds to Step S108, the determination unit 32 determines whether or not a tendency of the reversal DB is similar to that of a reference reversal DB (FIG. 8B) that is defined beforehand. The reference reversal DB in FIG. 8B may be created based on pieces of data of a plurality of users who have read the electronic document. In Step S108, as an example, the determination unit 32 calculates a total value and an average value of differences between lines of the reversal DB and the reference reversal DB and determines that the tendency of the reversal DB is similar to that of the reference reversal DB (FIG. 8B) when the calculated value is smaller than a threshold value that is defined beforehand.

When "Yes" is determined in Step S108, the flow proceeds to Step S30, and the determination unit 32 determines that the user has perused the electronic document. In addition, when "No" is determined in Step S108, the flow proceeds to Step S32, and the determination unit 32 determines that the user has not perused the electronic document.

As described above, in the third embodiment, the number of backward-directed eye movements for each of the lines is considered, so that the determination unit 32 may perform highly accurate perusing determination so as to consider a feature of the electronic document. In addition, even when the user who skims the electronic document pretends to peruse the electronic document by performing the backward-directed eye movements on purpose, it may not be determined that the user operation corresponds to perusing of the electronic document, by setting, as a criterion, whether or not the number of backward-directed eye movements for each of the lines is similar to the reference that is defined beforehand (reference reversal DB).

In the above-described third embodiment, the determination unit 32 obtains a variance (relative frequency) of the number of backward-directed eye movements in the each of the lines, in each of the reversal DB and the reference reversal DB, and may determine whether or not the tendency of the reversal DB is similar to that of the reference reversal DB, based on a total value and an average value of differences of variances (relative frequency) for each of the lines.

In the above-described third embodiment, the number of backward-directed eye movements is counted for each of the lines, so that it may be determined whether or not the user is perusing the electronic document even in the middle of reading of the electronic document by the user. Thus, the notification unit 34 may notify the user that it is desirable that the user peruses the electronic document in the middle of reading of the electronic document by the user, based on the determination result.

### [Fourth embodiment]

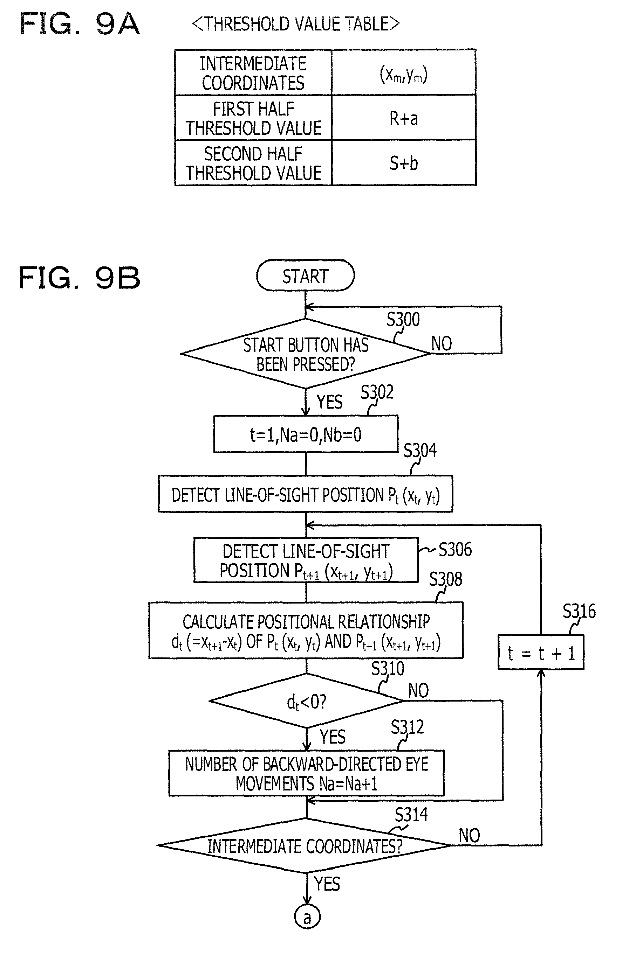

A fourth embodiment is described below with reference to FIGs. 9A to 10. A structure and the like of an information processing system according to the fourth embodiment are similar to those of the above-described information processing system 100 according to the first embodiment, but a method of determining whether or not the user has perused a document is different from the first embodiment.

FIG. 9A illustrates a data structure of a threshold value table to which the information processing device 10 refers. As illustrated in FIG. 9A, in the threshold value table, intermediate coordinates, a first half threshold value, and a second half threshold value are stored. The intermediate coordinates (xₘ, yₘ) indicates coordinates that are used to segment the first half and the second half of the electronic document. The first half threshold value "R+a" indicates a threshold value of the number of backward-directed eye movements from the beginning of the electronic document to the intermediate coordinates (xₘ, yₘ) (threshold value that is used to determine whether or not the user peruses the electronic document). Here, "R" indicates the number of lines in the first half of the electronic document, and "a" indicates the number of times in which it is highly probable that a backward-directed eye movement occurs in the first half of the electronic document. The second half threshold value "S+b" indicates a threshold value of the number of backward-directed eye movements from the intermediate coordinates (xₘ, yₘ) to the end of the electronic document (threshold value that is used to determine whether or not the user peruses the electronic document). Here, "S" indicates the number of lines in the second half of the electronic document, and "b" indicates the number of times in which it is highly probable that a backward-directed eye movement occurs in the second half of the electronic document. The values of "a" and "b" may be defined based on an experiment and a simulation, or may be defined based on a structural feature of the electronic document.

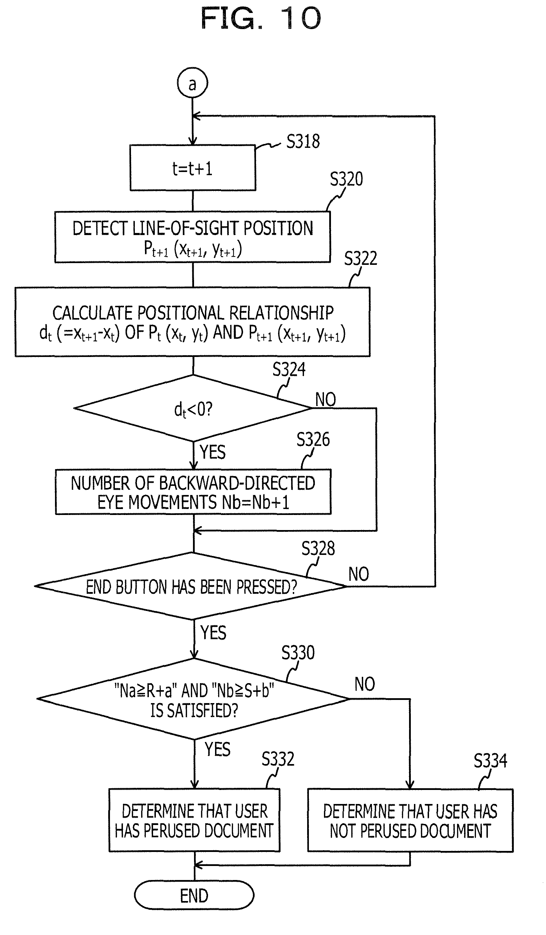

FIGs. 9B and 10 are flowcharts illustrating processing of the information processing device 10 according to the fourth embodiment. In the embodiment, when a start button is pressed by the user in Step S300, the eye tracking unit 30 sets, at 1, a value t that indicates a serial number of a line-of-sight position in Step S302. In addition, in Step S302, the eye tracking unit 30 sets, at 0, a value Na that indicates the number of backward-directed eye movements (first half), and sets, at 0, a value Nb that indicates the number of backward-directed eye movements (second half). After that, by repeating the processing and determination in Steps S304, S306 to S316, the return movement counting unit 31 counts the value Na of the number of backward-directed eye movements in the first half of the electronic document (S312). In addition, when "Yes" is determined in Step S314, that is, when the line-of-sight passes through the intermediate coordinates (xₘ, yₘ), the flow proceeds to Step S318 of FIG. 10.

After Step S318, by repeating the processing and determination in Steps S318 to S328, the return movement counting unit 31 counts the value Nb of the number of backward-directed eye movements in the second half of the electronic document (S312). In addition, when "Yes" is determined in Step S328, that is, when the end button is pressed by the user, the flow proceeds to Step S330.

When the flow proceeds to Step S330, the determination unit 32 determines whether or not the value Na is the first half threshold value "R+a" or more, and the value Nb is the second half threshold value "S+b" or more. When "Yes" is determined in Step S330, the flow proceeds to Step S332, and the determination unit 32 determines that the user has perused the electronic document. In addition, "No" is determined in Step S330, the flow proceeds to Step S334, and the determination unit 32 determines that the user has not perused the electronic document. The determination unit 32 may determine that the user has perused the electronic document when at least one of "Na≥R+a" and "Nb≥S+b" is satisfied in addition to a case in which both "Na≥R+a" and "Nb≥S+b" are satisfied.

As described above, in the fourth embodiment, the electronic document is segmented into the first half and the second half, and it is determined whether or not the user has perused the electronic document, based on whether or not the number of backward-directed eye movements in each of the areas satisfies a threshold value that is defined in each of the areas. Therefore, the perusing determination may be performed accurately as compared with a case in which the perusing determination is performed using one threshold value. For example, in a regular electronic document, in the second half, it is highly probable that a content that has been described in the first half is explained in detail, and a new content is less than the first half, so that it is conceivable that the number of backward-directed eye movements is reduced. Thus, by applying different threshold values to the first half and the second half of the electronic document, appropriate perusing determination may be performed in which features of the document in the first half and the second half are considered.

In the above-described fourth embodiment, the electronic document is segmented into the first half and the second half, but the embodiment is not limited to such an example. The electronic document may be segmented into three or more, and a threshold value of the number of backward-directed eye movements may be set for each of the areas.

In the above-described fourth embodiment, a device error of the line-of-sight detection device 16 is considered, and the intermediate coordinates may be defined within the range.

In the above-described fourth embodiment, the case is described in which the different threshold values are applied to the first half and the second half, but the embodiment is not limited to such a case. In the first half, processing that is similar to the processing in one of the first to third embodiments may be executed, and in the second half, processing that is similar to the processing in one of the first to third embodiments and different from the processing in the first half may be executed.

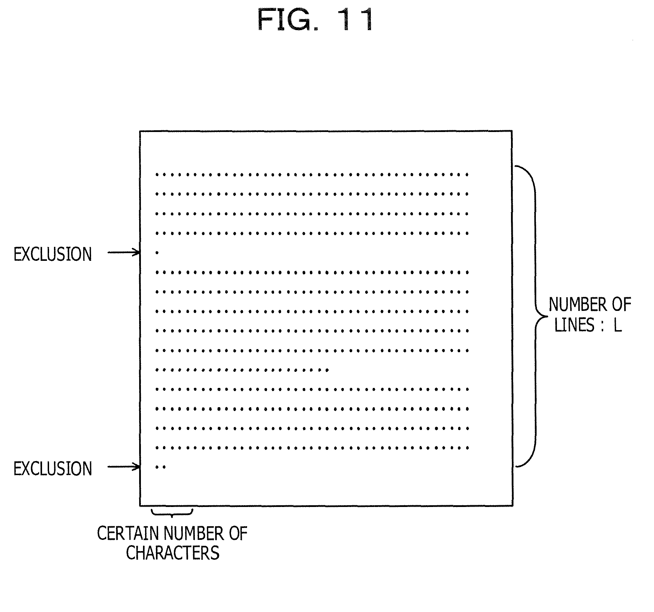

In each of the above-described embodiments, the number of characters in each of the lines may be considered. For example, from among the lines, there is a line having a small number of characters (line having the number of characters is smaller than the certain number of characters as illustrated by an arrow in FIG. 11). In such a case, the line having a small number of characters is removed from the L lines, and a threshold value of the number of backward-directed eye movements may be defined. In addition, in the second embodiment, backward-directed eye movement in a line having a small number of characters may not be counted. In addition, there is a case in which the line-of-sight is moved immediately after a return sweep occurs in order to set the line-of-sight to a correct position (beginning of the line). Thus, the backward-directed eye movement in an area around the beginning of the line immediately after the line change occurs may not be counted.

In each of the above-described embodiments, an occurrence tendency of a backward-directed eye movement may be predicted, and a threshold value may be determined based on whether what kind of document the user is familiar with in the past, or a degree of difficulty for the user of electronic document. In this case, a threshold value is defined for each user.

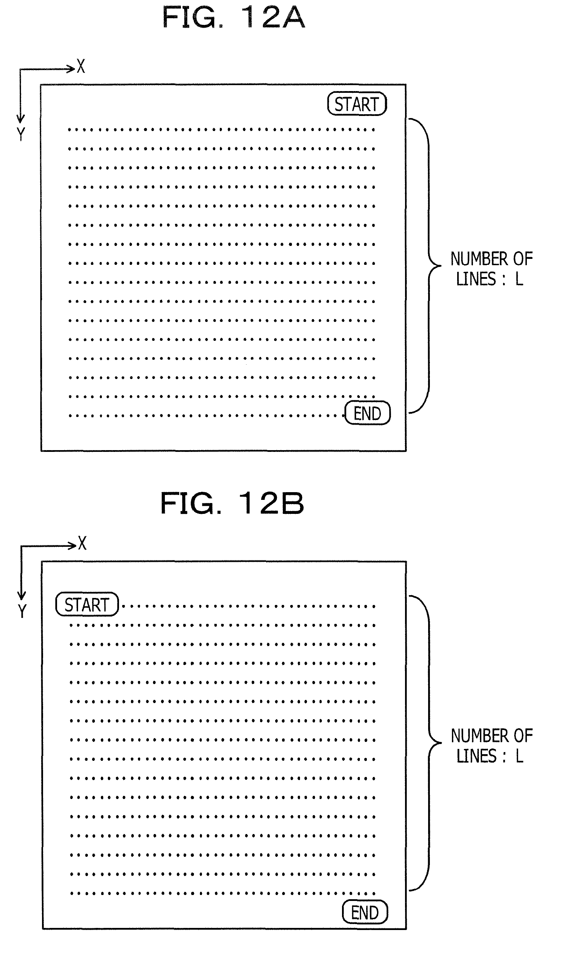

In each of the above-described embodiments, the case is described in which the start button and the end button are arranged as illustrated in FIG. 4, but the embodiment is not limited to such a case. For example, the start button and the end button may be arranged as illustrated in FIG. 12A. In addition, the start button and the end button may be arranged as illustrated in FIG. 12B. Here, when the arrangement as illustrated in FIG. 12B is employed, for example, as the threshold value that is used in Step S28 of FIG. 5, which is described in the first embodiment, it is desirable that "L" that is obtained by subtracting "1" from "L+1" is used.

In each of the above-described embodiments, the case is described in which the electronic document corresponds to horizontal writing, but the embodiment is not limited to such a case, and the electronic document may correspond to vertical writing. Even in the case of the vertical writing, it may be accurately determined whether or not the user has perused the electronic document by executing processing that is similar to the processing in each of the above-described embodiments.

The processing that is described in each of the above-described embodiments may be executed by a further information processing device (server or the like) that is connected to the information processing device 10 through a network.

The above-described processing function may be obtained by a computer. In that case, there is provided a program in which a processing content of a function that is to be included in a processing device is described. By executing the program through the computer, the above-described processing function is achieved on the computer. The program in which the processing content is described may be recorded to a computer readable recording medium (here, carrier waves are excluded).

When the program is distributed, for example, the program is sold in the form of a portable recording medium such as a digital versatile disc (DVD) and a compact disc read only memory (CD-ROM) to which the program is recorded. In addition, the program may be stored in a storage device of a server computer, and the program may be transferred from the server computer to a further computer through a network.

The computer that executes a program stores, for example, a program that is recorded to the portable recording medium or a program that is transferred from the server computer, in the storage device of the computer. In addition, the computer reads the program from the storage device, and executes processing in accordance with the program. The computer may read the program from the portable recording medium directly, and execute the processing in accordance with the program. In addition, the computer may execute the processing in accordance with the read program each time the program is transferred from the server computer.

The above-described embodiments are preferable examples. However, the embodiments are not limited such examples, and various modifications may be made without departing from the gist of the present disclosure.

## Claims

1. A perusing determination device, includes a computer processor, the device comprising:
an eye tracking unit configured to detect a movement direction of line-of-sight of a user who reads a document;
a return movement counting unit configured to count a first number of times in which the movement direction of the line-of-sight is reversed from a direction in which the document is to be read; and
a determination unit configured to determine whether or not the user peruses the document, based on the first number and a second number of times in which the movement direction of the line-of-sight is reversed that is to be satisfied at a time of perusing and is defined based on a feature of the document.

2. The perusing determination device according to claim 1,
wherein the return movement counting unit is configured to count the first number is reversed by a distance that is shorter than a length of a line of the document.

3. The perusing determination device according to any preceding claim,
wherein the second number is to be satisfied at the time of perusing is a value that is defined based on a number of lines that are included in the document.

4. The perusing determination device according to claim 3,
wherein the second number is to be satisfied at the time of perusing is a value that is defined based on the number of lines that includes a certain number of characters or more, from among the lines that are included in the document.

5. The perusing determination device according to any preceding claim,
wherein the return movement counting unit is configured to count the first number for each of the lines in the document,
wherein the determination unit is configured to determine whether or not the user peruses the document, based on a comparison result between an occurrence tendency of the second number in each of the lines, which is to be satisfied at the time of perusing and is defined based on the feature of the document, and the counting result of the return movement counting unit.

6. A perusing determination method comprising:
detecting a movement direction of line-of-sight of a user who reads a document;
counting a first number of times in which the movement direction of the line-of-sight is reversed from a direction in which the document is to be read; and
determining whether or not the user peruses the document, based on the first number and a second number of times in which the movement direction of the line-of-sight is reversed that is to be satisfied at a time of perusing and is defined based on a feature of the document.

7. The method according to claim 6,
wherein in the counting, the first number of times in which the movement direction of the line-of-sight is reversed by a distance that is shorter than a length of a line of the document is counted.

8. The method according to any preceding method claim,
wherein the second number is to be satisfied at the time of perusing is a value that is defined based on a number of lines that are included in the document.

9. The method according to claim 8,
wherein the second number is to be satisfied at the time of perusing is a value that is defined based on the number of lines that includes a certain number of characters or more, from among the lines that are included in the document.

10. The method according to any preceding method claim,
wherein, in the counting, the first number is counted for each of the lines in the document,
wherein, in the determining, it is determined whether or not the user peruses the document, based on a comparison result between an occurrence tendency of the second number in each of the lines, which is to be satisfied at the time of perusing and is defined based on the feature of the document, and the counting result of the counting.

11. A computer-readable storage medium storing a perusing determination program that causes a computer to execute a process, the process comprising:
detecting a movement direction of line-of-sight of a user who reads a document;
counting a first number of times in which the movement direction of the line-of-sight is reversed from a direction in which the document is to be read; and
determining whether or not the user peruses the document, based on the first number and a second number of times in which the movement direction of the line-of-sight is reversed that is to be satisfied at a time of perusing and is defined based on a feature of the document.
